# EUROPÄISCHE PATENTANMELDUNG

(11) **EP 3 091 356 A1**
(43) Veröffentlichungstag der Anmeldung: **09.11.2016**
(21) Anmeldenummer: 16168099.6
(22) Anmeldetag: 03.05.2016
(51) Int. Cl.: G01N 33/28

(54) **VERFAHREN ZUR BESTIMMUNG EINER DIE KLOPFFESTIGKEIT CHARAKTERISIERENDEN KENNGRÖSSE EINES KRAFTSTOFFS SOWIE ENTSPRECHENDE PRÜFANORDNUNG**

(30) Priorität: 04.05.2015 DE 102015106881
(71) Anmelder: Rofa Laboratory & Process Analyzers, 3420 Kritzendorf (AT)
(72) Erfinder: Huber, Prof. Dr. Karl, 85072 Eichstätt (DE); Hauber, Johann, 86633 Neuburg (DE)
(74) Vertreter: Baudler, Ron

(57) **Zusammenfassung**

Die Erfindung betrifft ein Verfahren zur Bestimmung einer die Klopffestigkeit charakterisierenden Kenngröße eines Kraftstoffs mit Hilfe eines, wenigstens einen Zylinder (1) aufweisenden, Prüfmotors (2), wobei der Kraftstoff im Zuge des Verfahrens innerhalb des Zylinders (1) verbrannt wird und der bei der Verbrennung entstehende Zylinderdruck mit Hilfe eines Drucksensors (4) detektiert wird, wobei ein Drucksensor (4) mit einer linearen Druck-Ausgangssignal-Kennlinie zum Einsatz kommt. Erfindungsgemäß wird vorgeschlagen, dass basierend auf dem Ausgangssignals des Drucksensors (4) eine die Klopffestigkeit des Kraftstoffs charakterisierende Kenngröße berechnet wird, wobei die Berechnung auf Basis eines mathematischen Modells erfolgt, welches die Abweichung der Druck-Ausgangssignal-Kennlinie des zum Einsatz kommenden Drucksensors (4) von der Druck-Ausgangssignal-Kennlinie eines in der Norm ASTM D2699 vorgeschriebenen Pickup Sensors (5) berücksichtigt. Darüber hinaus wird eine Prüfanordnung zur Bestimmung der Klopffestigkeit eines Kraftstoffs vorgeschlagen.

## Beschreibung

Die vorliegende Erfindung betrifft ein Verfahren zur Bestimmung einer die Klopffestigkeit charakterisierenden Kenngröße eines Kraftstoffs mit Hilfe eines, wenigstens einen Zylinder aufweisenden, Prüfmotors, wobei der Kraftstoff im Zuge des Verfahrens innerhalb des Zylinders verbrannt wird und der bei der Verbrennung entstehende Zylinderdruck mit Hilfe eines Drucksensors detektiert wird, wobei ein Drucksensor mit einer linearen Druck-Ausgangssignal-Kennlinie zum Einsatz kommt.

Ferner wird eine Prüfanordnung zur Bestimmung einer die Klopffestigkeit charakterisierenden Kenngröße eines Kraftstoffs mit einem Prüfmotor, der wenigstens einen Zylinder und einen Drucksensor mit einer linearen Druck-Ausgangssignal-Kennlinie aufweist, vorgeschlagen, wobei mit Hilfe des Drucksensors der bei der Verbrennung des Kraftstoffs im Zylinder herrschende Zylinderdruck detektierbar ist.

Die Klopffestigkeit stellt eines der wichtigsten Qualitätsmerkmale von Ottokraftstoffen dar, zu deren Bestimmung genormte motorische Prüfverfahren angewandt werden. Diese Verfahren wurden vor über 80 Jahren entwickelt und sind bis heute nahezu unverändert weltweit in den Kraftstoffprüflaboren im Einsatz.

Die Endlichkeit fossiler Kraftstoffe führt im Bereich der Kraftfahrzeuge zu einer immer stärkeren Diversifizierung der Antriebsarten und der Energieträger. Insbesondere bei den flüssigen Kohlenwasserstoffen ist davon auszugehen, dass weltweit das Angebot an unterschiedlichen Kraftstoffen mit biogenen Beimischungen weiter zunehmen wird. In gleichem Maße wächst die Bedeutung der Kraftstoffprüfung, da die Kraftstoffqualität als Voraussetzung für eine effiziente und emissionsarme Verbrennung anzusehen ist.

Im Ottomotor ist die Klopffestigkeit des Kraftstoffs ein Qualitätsmerkmal von höchster Bedeutung, da sie über die mögliche Verdichtung bereits bei der konstruktiven Auslegung sowie über den Zündwinkeleingriff der Klopfregelung im Betrieb des Motors direkten Einfluss auf den erreichbaren Wirkungsgrad hat. Die Notwendigkeit, Ottokraftstoffe auf ihre Klopffestigkeit zu prüfen, wurde aufgrund von Motorschäden bereits in den 1930er-Jahren erkannt und ein Prüfverfahren zur Bestimmung der Klopffestigkeit am Markt etabliert.

Die die Klopffestigkeit charakterisierenden Kenngrößen (Oktanzahlen) werden gemäß den Normen ASTM D2699 (Research-Oktanzahl: ROZ) und D2700 (Motor-Oktanzahl: MOZ) beziehungsweise deren europäischen Entsprechungen EN-ISO 5163 und EN-ISO 5164 an einem genormten Prüfmotor durch sogenannte Eingabelung mit Gemischen aus Isooktan und n-Heptan bestimmt.

In der Regel kommt in diesem Zusammenhang ein von Waukesha entwickelter Cooperative-Fuel-Research-Motor (CFR-Motor) zum Einsatz. Hierbei handelt es sich um einen Einzylinder-Prüfmotor mit Zweiventiltechnik und seitlicher Zündkerzenlage. Er zeichnet sich vor allem durch das im Betrieb veränderliche Verdichtungsverhältnis aus. Der Motor verfügt über äußere Gemischbildung mittels Vergaser sowie Luftvorwärmung und des Weiteren beim MOZ-Verfahren über eine Gemischheizung. Die Oktanzahl eines Kraftstoffs wird normgerecht durch Eingabelung mit Referenzkraftstoffen bestimmt. Hierzu ist die ordnungsgemäße Funktion des Prüfmotors mithilfe von toluolhaltigen Einstellkraftstoffen (TSF) zu prüfen, wobei unter anderem die Ansaugtemperatur einzustellen ist.

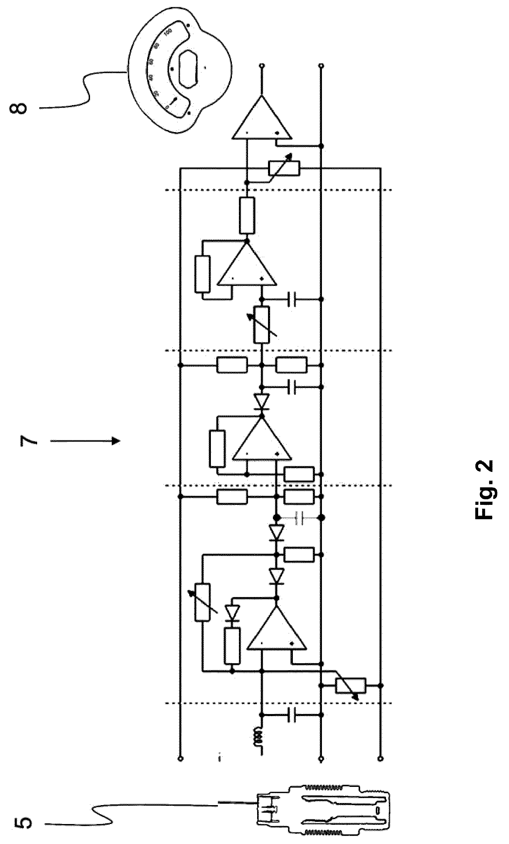

Um die Oktanzahl eines unbekannten Kraftstoffs zu bestimmen, ist das Luftverhältnis durch den Bediener so einzustellen, dass das bei der Verbrennung auftretende Klopfen maximal ist. Zum Eingabeln werden zwei Kraftstoffgemische mit bekannter Oktanzahl benötigt. Verwendet werden hierzu für Oktanzahlen unter 100 Zweikomponentengemische aus dem klopffesteren und namensgebenden Isooktan (2,2,4-Trimethylpentan) und dem klopffreudigeren n-Heptan. Von diesen beiden Gemischen muss mit dem einen ein höherer, mit dem anderen ein geringerer Ausschlag am Knockmeter (= Drehspulinstrument, siehe hierzu auch Figur 2 sowie die folgende Beschreibung) erzielt werden, um die Oktanzahl der dazwischenliegenden Kraftstoffprobe linear zu interpolieren.

Problematisch ist hier jedoch unter anderem die Bestimmung von ROZ-Werten über 100, da hierzu verbleite Referenzkraftstoffe (Isooktan mit Zugaben von Bleitetraethyl) vorgesehen sind. Sie bergen ein erhebliches Gesundheitsrisiko für das Bedienpersonal und sind deswegen in Europa nur eingeschränkt verfügbar. Deshalb erfolgt das Eingabein in vielen Laboren entgegen der Norm mit TSF-Kraftstoffen, die eigentlich nur zur Eichpunktfindung herangezogen werden sollten.

Da die Knockmeteranzeige die wichtigste Größe bei der Bestimmung der ROZ und MOZ ist, wird nachfolgend genauer auf die Signalerfassung mit dem sogenannten Pickup Sensor, die Signalanalyse im so genannten Detonationmeter sowie die Anzeige der Klopfstärke am Knockmeter, wie sie in den oben genannten Normen vorgesehen sind, eingegangen.

Der mit dem Zylinderraum des zum Einsatz kommenden Prüfmotors verbundene Pickup Sensor besteht im Wesentlichen aus einem mit einer Spule umwickelten magnetostriktiven Stab. Durch Deformation des Stabs infolge des Zylinderdrucks wird eine Spannung in der Spule induziert. Diese ist die einzige Messgröße, die in die Auswertelogik des Detonationmeters eingeht (das Detonationmeter ist eine Analogschaltung, die die Aufgabe hat, das Pickup-Signal zu verarbeiten und daraus eine Spannung zu erzeugen, deren Größe ein Maß für die Klopfstärke ist und vom Knockmeter zur Anzeige gebracht wird).

Untersuchungen haben nun gezeigt, dass sich das vom Pickup Sensor ausgegebene Ausgangssignal nicht über den gesamten relevanten Messbereich proportional zum tatsächlichen Zylinderdruck verhält, so dass die am Knockmeter angezeigten Werte nicht immer ein korrektes Maß für die Klopffestigkeit des analysierten Kraftstoffs darstellen.

Ein alternatives Verfahren zur Bestimmung einer die Klopffestigkeit charakterisierenden Kenngröße ist daher in der WO 2009/130254 A1 beschrieben, bei dem der Zylinderdruck mit einem Drucksensor gemessen wird, dessen Ausgangssignal sich proportional zum Zylinderdruck verhält (der Drucksensor hat also eine lineare Druck-Ausgangssignal-Kennlinie). Die ermittelten Drucksignale werden schließlich statistisch ausgewertet, um letztendlich eine Kenngröße zu generieren, die eine zuverlässige Bewertung der tatsächlichen Klopffestigkeit des jeweiligen Kraftstoffs ermöglicht.

Nachteilig ist jedoch, dass die gemäß WO 2009/130254 A1 ermittelten Kenngrößen nicht mehr mit den gemäß den oben genannten Normen ermittelten Kenngrößen übereinstimmen.

Aufgabe der vorliegenden Erfindung ist es, diesem Nachteil zu begegnen.

Die Aufgabe wird gelöst durch ein Verfahren sowie eine Prüfanordnung mit den Merkmalen der unabhängigen Patentansprüche.

Erfindungsgemäß zeichnet sich das Verfahren dadurch aus, dass basierend auf dem Ausgangssignals des Drucksensors eine die Klopffestigkeit des mit Hilfe des zum Einsatz kommenden Prüfmotors analysierten Kraftstoffs charakterisierende Kenngröße berechnet wird. Die Berechnung erfolgt hierbei auf Basis eines mathematischen Modells, welches die Abweichung der Druck-Ausgangssignal-Kennlinie des zum Einsatz kommenden Drucksensors von der Druck-Ausgangssignal-Kennlinie eines in der Norm ASTM D2699 und D2700 vorgeschriebenen Pickup Sensors berücksichtigt (wobei es sich bei der genannten Norm im Rahmen der vorliegenden Erfindung um die Version handelt, die zum Prioritätstag der vorliegenden Anmeldung gültig ist; ASTM = "American Society for Testing and Materials").

Mit anderen Worten dient also das Ausgangssignal des Drucksensors (welches sich anders als das vom genannten Pickup Sensor erzeugte Signal proportional zum tatsächlich innerhalb des Zylinders vorherrschenden Druck verhält) als Eingangsgröße eines mathematischen Modells verwendet, mit dessen Hilfe schließlich eine die Klopffestigkeit des Kraftstoffs charakterisierende Kenngröße berechnet wird.

Die Kenngröße hat schließlich denselben Betrag, den sie hätte, wenn der Prüfmotor mit dem genannten Pickup Sensor ausgestattet wäre und das vom Pickup Sensor gelieferte Signal entsprechend einer der oben genannten Normen weiterverarbeitet würde. Im Ergebnis erhält man mit dem erfindungsgemäßen Verfahren also eine Kenngröße, insbesondere in Form einer Oktanzahl, welche die Klopffestigkeit des analysierten Kraftstoffs widerspiegelt, wobei im Gegensatz zu den oben genannten Normen ein Drucksensor zum Einsatz kommt, dessen Ausgangssignal sich proportional zum anliegenden Zylinderdruck verhält.

Somit ist es schließlich möglich, mit Hilfe der in der WO 2009/130254 A1 beschriebenen Prüfanordnung die Klopffestigkeit eines Kraftstoffs zu bestimmen, wobei die ermittelte Klopffestigkeit mit der Klopffestigkeit übereinstimmt, die man bei Einhaltung einer der oben genannten Normen erhalten würde (ob es sich bei der Klopffestigkeit nun um die ROZ oder MOZ handelt, hängt im Wesentlichen von der Einstellung diverserer Parameter des Prüfmotors, wie beispielsweise dessen Drehzahl ab und hat damit keinen Einfluss auf die Anwendbarkeit der vorliegenden Erfindung).

Vorzugsweise wird mit Hilfe des erfindungsgemäßen Verfahrens bzw. der erfindungsgemäßen Prüfanordnung also die ROZ oder die MOZ bestimmt, wobei das mathematische Modell derart gewählt wird, dass der Betrag der jeweiligen Kenngröße dem Betrag entspricht, den man erhalten würde, wenn man eine der obigen Normen einhalten würde.

Insbesondere ist es vorteilhaft, wenn das mathematische Modell eine Übertragungsfunktion umfasst, mit deren Hilfe aus dem Ausgangssignal des Drucksensors die die Klopffestigkeit charakterisierende Kenngröße berechnet wird. Die Übertragungsfunktion dient also der Umrechnung des Ausgangssignals des Drucksensors in die entsprechende Kenngröße (ROZ oder MOZ), wobei die Übertragungsfunktion derart beschaffen ist, dass die erhaltene Kenngröße betragsmäßig der Kenngröße entspricht, die unter Einhaltung einer der genannten Normen ermittelt werden würde (d. h. bei Einsatz eines Pickup Sensors).

Vorteilhaft ist es, wenn die Übertragungsfunktion als Differentialgleichung ausgebildet ist oder eine solche umfasst. Insbesondere ist es hierbei zweckmäßig, dass bestimmte Signalanteile des Ausgangssignals des Drucksensors der Differentialgleichung als Eingangsgröße zugeführt werden. Beispielsweise ist es denkbar, dass nur das Signal bzw. nur die Signalanteile Verwendung finden, die während des maximalen Zylinderdrucks vom Drucksensor geliefert werden.

Vorteilhaft ist es zudem, wenn die Differentialgleichung zeitliche Ableitungen unterschiedlicher Ordnung des Ausgangssignals des Drucksensors beziehungsweise des hieraus ermittelten Zylinderdrucks umfasst. Insbesondere die erste Ableitung, d.h. die Änderung des Drucks pro Zeit, liefert Informationen, die Rückschlüsse auf die Klopffestigkeit des analysierten Kraftstoffs erlauben.

Ebenso ist es vorteilhaft, wenn die zeitlichen Ableitungen innerhalb der Differentialgleichung unterschiedlich stark gewichtet werden. Beispielsweise wäre es denkbar, alle oder einzelne Ableitungen mit einem Faktor zu multiplizieren, wobei die jeweiligen Faktoren zumindest teilweise unterschiedliche Beträge aufweisen. Je nach der Höhe des Betrags des ermittelten Zylinderdrucks, d.h. je nach der Höhe des Betrags des Ausgangssignals des Drucksensors, erfolgt somit eine unterschiedlich starke Korrektur des Ausgangssignals, so dass im Ergebnis eine Kenngröße ermittelt wird, deren Betrag sich nicht von der Kenngröße unterscheidet, die man erhalten würde, wenn man dieselbe Kraftstoffprobe gemäß einer der obigen Normen analysieren würde.

Auch ist es äußert vorteilhaft, wenn mit Hilfe des mathematischen Modells der in der Norm ASTM D2699 bzw. D2700 vorgeschriebenen Pickup Sensor simuliert wird, indem das Ausgangssignal des Drucksensors derart korrigiert wird, dass das korrigierte Signal im Wesentlichen dem Ausgangssignal des in der Norm ASTM D2699 bzw. D2700 vorgeschriebenen Pickup Sensors bei gleichem Zylinderdruck entspricht. Das korrigierte Ausgangssignal könnte schließlich einem in den genannten Normen beschriebenen Detonationmeter zugeführt werden, das schließlich ein vom Ausgangssignal abhängiges Signal an das ebenfalls in den genannten Normen genannten Knockmeter weitergibt. Das Knockmeter zeigt schließlich die gewünschte Klopfstärke an, die zur Eingabelung mittels linearer Interpolation die Oktanzahl liefert, die man bei vollständiger Einhaltung einer der oben genannten Normen erhalten würde (d.h. bei Einsatz eines Pickup-Sensors anstelle des erfindungsgemäßen Drucksensors mit linearer Druck-Ausgangssignal-Kennlinie).

Ebenso bringt es Vorteile mit sich, wenn mit Hilfe des mathematischen Modells die in der Norm ASTM D2699 und D2700 beschriebene Messkette aus Pickup Sensor, Detonationmeter und Knockmeter simuliert wird. Das mathematische Modell bildet also die genannte Messkette wieder, wobei der durch den Drucksensor ermittelte Zylinderdruck als Eingangsgröße für die simulierte Messkette dient, um im Ergebnis die entsprechende Oktanzahl (ROZ oder MOZ) zu berechnen.

Besondere Vorteile bringt es mit sich, wenn das Ausgangssignal des Drucksensors mit Hilfe eines Tiefpassfilters gefiltert wird, so dass die Frequenzen des Ausgangssignals entfernt werden, deren Betrag über einem bestimmten Grenzwert liegen, wobei die die Klopffestigkeit des Kraftstoffs charakterisierende Kenngröße auf Basis des gefilterten Ausgangssignals berechnet wird.

Besonders vorteilhaft ist es, wenn als Drucksensor ein piezoelektrischer Drucksensor zum Einsatz kommt, da sich derartige Sensoren durch eine sehr hohe Eigenfrequenz und eine exzellente Linearität auszeichnen.

Vorteilhaft ist es, wenn die Auswertung des Ausgangssignals des Drucksensors unter Einsatz eines künstlichen neuronalen Netzes erfolgt. Mit Hilfe des neuronalen Netzes kann auf Basis eines entsprechenden Lerndatensatzes (Zusammenhang zwischen Ausgangssignal des Drucksensors und der jeweiligen unter Berücksichtigung einer der obigen Normen ermittelten Oktanzahl der zu analysierenden Kraftstoffprobe, wobei eine Vielzahl unterschiedlicher Kraftstoffarten analysiert werden) ein Modell entwickelt werden, auf dessen Basis schließlich eine Umrechnung des Ausgangssignals des Drucksensors (das bei der Verbrennung einer zu analysierenden Kraftstoffprobe detektiert wird) in eine entsprechende Oktanzahl (ROZ oder MOZ) erfolgen kann.

Schließlich wird eine Prüfanordnung vorgeschlagen, mit deren Hilfe sich das beschriebene Verfahren durchführen lässt.

Die Prüfanordnung dient der Bestimmung einer die Klopffestigkeit charakterisierenden Kenngröße eines Kraftstoffs mit einem Prüfmotor, wobei der Prüfmotor wenigstens einen Zylinder und einen Drucksensor mit einer linearen Druck-Ausgangssignal-Kennlinie aufweist, und wobei mit Hilfe des Drucksensors der bei der Verbrennung des Kraftstoffs im Zylinder herrschende Zylinderdruck detektierbar ist.

Erfindungsgemäße wird vorgeschlagen, dass die Prüfanordnung eine Auswerteeinheit umfasst, mit deren Hilfe (bzw. mit Hilfe einer in der Auswerteeinheit hinterlegten Software) basierend auf dem Ausgangssignal des Drucksensors eine die Klopffestigkeit des Kraftstoffs charakterisierende Kenngröße berechenbar ist, wobei die Berechnung auf Basis eines mathematischen Modells erfolgt, welches die Abweichung der Druck-Ausgangssignal-Kennlinie des zum Einsatz kommenden Drucksensors von der Druck-Ausgangssignal-Kennlinie eines in der Norm ASTM D2699 bzw. D2700 vorgeschriebenen Pickup Sensors berücksichtigt.

Generell kann die Auswerteeinheit hierbei ausgebildet sein, das Verfahren gemäß bisheriger Beschreibung auszuführen, wobei einzelne Merkmale in beliebiger Kombination verwirklicht werden können (soweit dies nicht zu Widersprüchen führt).

Insbesondere kann die Prüfanordnung somit auch einen Tiefpassfilter oder einen als Piezodrucksensor ausgebildeten Drucksensor aufweisen. Auf den Einsatz des in der Norm ASTM D2699 bzw. D2700 definierten Pickup Sensors kann somit verzichtet werden.

Weitere Aspekte der Erfindung sind nachfolgend beschrieben. Es zeigen, jeweils schematisch:
- **Figur 1**: einen Prüfmotor zur Bestimmung der Klopffestigkeit eines Kraftstoffs, wie er beispielsweise gemäß der Norm ASTM D2699 (EN ISO 5163) bzw. D2700 (EN ISO 5164) zum Einsatz kommt,
- **Figur 2**: eine schematische Darstellung der in der Norm ASTM D2699 (EN ISO 5163) bzw. D2700 (EN ISO 5164) beschriebenen Messkette, und
- **Figur 3**: eine erfindungsgemäße Prüfanordnung zur Durchführung des erfindungsgemäßen Verfahrens.

Die Ermittlung der Oktanzahlen wird heute weltweit empirisch und nach genormten Verfahren in den Laboren der Kraftstoffproduzenten durchgeführt. Zum Einsatz kommen dabei spezielle Einzylinder-Prüfmotoren, deren Verdichtungsverhältnis variable ist und auf die jeweilige Kraftstoffqualität eingestellt werden kann.

Ziel ist es, den zu prüfenden Kraftstoff hinsichtlich seiner Klopfintensität mit Kraftstoffen bekannter Oktanzahlen zu vergleichen und ggf. durch Interpolation die Oktanzahlen zu ermitteln. Willkürlich wurde in der Norm Iso-Oktan die Oktanzahl 100 und n-Heptan die Oktanzahl 0 zugeordnet. Durch Mischen dieser Komponenten kann jeweils ein Kraftstoff hergestellt werden, der die gleiche Klopfintensität wie der zu prüfende Kraftstoff aufweist. Die gesuchte Oktanzahl entspricht dann dem volumetrischen Anteil an Iso-Oktan der Kraftstoffmischung. Hinsichtlich der Prüfbedingungen wird zwischen MOZ und ROZ unterschieden, wobei alle anderen Verfahrensschritte übereinstimmen und auch dieselbe Messtechnik und derselbe Prüfmotor 2 verwendet werden.

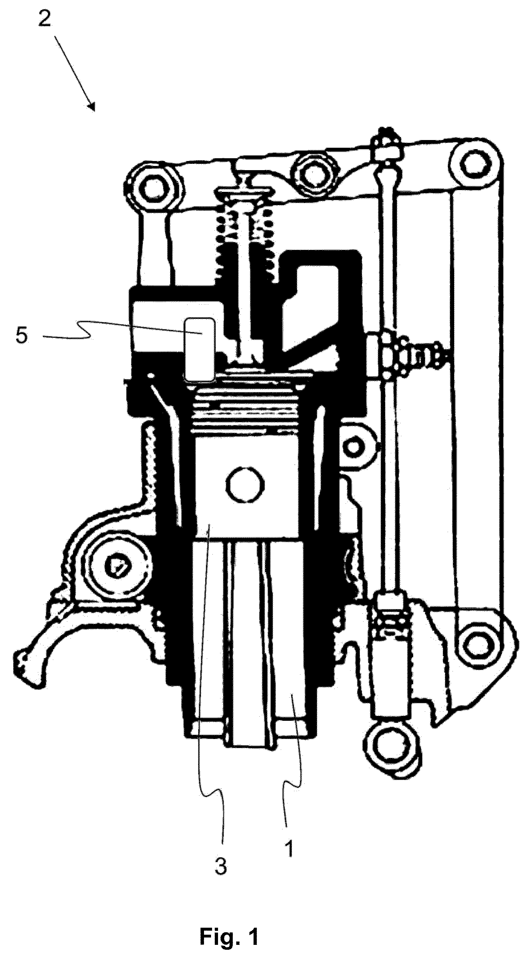

Figur 1 zeigt einen Prüfmotor 2 zur Bestimmung der Klopffestigkeit eines Kraftstoffs, wie er beispielsweise gemäß der Norm ASTM D2699 bzw. D2700 (Bestimmung der ROZ bzw. MOZ) zum Einsatz kommt.

Das Maß der Klopfintensität wird hierbei über einen elektrischen Sensor (Pickup Sensor 5), der in den Brennraum des Motors eingeschraubt ist (Fig. 1), erzeugt und über eine analoge Schaltung, dem so genannten Detonationmeter 7 auf ein Zeigerinstrument (Knockmeter 8) zur Anzeige gebracht (Fig. 2).

Aus eigenen Untersuchungen ist bekannt, dass das vom Pickup Sensor 5 gelieferte Signal nicht proportional zum Zylinderdruck verläuft, so dass mit dem genannten Verfahren eine Genauigkeit von höchstens +/- 0,2 Oktanzahlen erreicht werden kann.

In der WO 2009/130254 A1 ist ferner ein Verfahren beschrieben, bei dem der Zylinderdruck mit einem Drucksensor 4 gemessen wird, dessen Ausgangssignal sich proportional zum Zylinderdruck verhält (der Drucksensor 4 hat also eine lineare Druck-Ausgangssignal-Kennlinie). Die ermittelten Drucksignale werden statistisch ausgewertet, um letztendlich eine Kenngröße zu generieren, die eine zuverlässige Bewertung der tatsächlichen Klopffestigkeit des jeweiligen Kraftstoffs ermöglicht.

Der Nachteil an dem darin beschrieben Verfahren ist jedoch, dass die ermittelten Kennwerte nicht mit den Oktanzahlen übereinstimmen, die man bei Einhaltung einer der oben genannten Normen erhält.

Um nun mit der in der WO 2009/130254 A1 beschriebenen Prüfanordnung auch eine Oktanzahl bestimmen zu können, die mit der Oktanzahl übereinstimmt, die bei Einhaltung einer der genannten Normen ermittelt werden würde, wird nun vorgeschlagen, dass basierend auf dem Ausgangssignal des zum Einsatz kommenden Drucksensors 4 (der eine lineare Druck-Ausgangssignal-Kennlinie aufweist) eine die Klopffestigkeit des Kraftstoffs charakterisierende Kenngröße berechnet wird, wobei die Berechnung auf Basis eines mathematischen Modells erfolgt, welches die Abweichung der Druck-Ausgangssignal-Kennlinie des zum Einsatz kommenden Drucksensors 4 von der Druck-Ausgangssignal-Kennlinie eines in der Norm ASTM D2699 bzw. D2700 vorgeschriebenen Pickup Sensors 5 berücksichtigt.

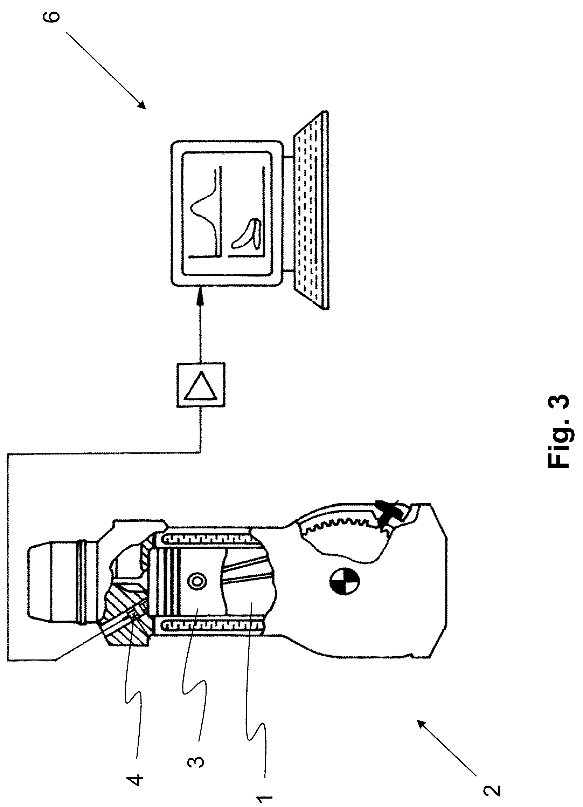

Es kommt also erfindungsgemäß ein Prüfmotor 2 zum Einsatz, wie er schematisch in Figur 3 gezeigt ist, wobei der Prüfmotor 2 einen Zylinder 1, einen innerhalb des Zylinders 1 geführten Kolben 3 und einen Drucksensor 4 zur Detektion des bei der Verbrennung eines Kraftstoffs entstehenden Zylinderdrucks aufweist (wobei der Drucksensor 4 derart ausgebildet ist, dass sich dessen Ausgangssignal proportional zum Zylinderdruck verhält).

Ferner steht der Drucksensor 4 mit einer Auswerteeinheit 6 (z.B. einem Personal Computer) in Verbindung, auf dem eine Auswertesoftware installiert ist, mit deren Hilfe das Ausgangssignal nach dem oben beschriebenen erfindungsgemäßen Verfahren auswertbar und in eine Oktanzahl überführbar ist.

Der Kern der Erfindung liegt somit generell darin, mit einer Prüfanordnung, die keinen der Norm ASTM D2699 (EN ISO 5163) bzw. D2700 (EN ISO 5164) entsprechenden Pickup Sensor 5 aufweist, eine Oktanzahl zu bestimmen, die der Oktanzahl entspricht, die man erhalten würde, wenn man die entsprechende Kraftstoffprobe gemäß einer der obigen Normen und somit unter Verwendung eines Pickup Sensors 5 analysieren würde.

### Bezugszeichenliste

- 1: Zylinder
- 2: Prüfmotor
- 3: Kolben
- 4: Drucksensor
- 5: Pickup Sensor
- 6: Auswerteeinheit
- 7: Detonationmeter
- 8: Knockmeter

## Patentansprüche

1. Verfahren zur Bestimmung einer die Klopffestigkeit charakterisierenden Kenngröße eines Kraftstoffs mit Hilfe eines, wenigstens einen Zylinder (1) aufweisenden, Prüfmotors (2), wobei der Kraftstoff im Zuge des Verfahrens innerhalb des Zylinders (1) verbrannt wird und der bei der Verbrennung entstehende Zylinderdruck mit Hilfe eines Drucksensors (4) detektiert wird, wobei ein Drucksensor (4) mit einer linearen Druck-Ausgangssignal-Kennlinie zum Einsatz kommt,
**dadurch gekennzeichnet,**
**dass** die Kenngröße basierend auf dem Ausgangssignal des Drucksensors (4) berechnet wird, wobei die Berechnung auf Basis eines mathematischen Modells erfolgt, welches die Abweichung der Druck-Ausgangssignal-Kennlinie des zum Einsatz kommenden Drucksensors (4) von der Druck-Ausgangssignal-Kennlinie eines in der Norm ASTM D2699 vorgeschriebenen Pickup Sensors (5) berücksichtigt.

2. Verfahren gemäß dem vorangegangenen Anspruch, **dadurch gekennzeichnet, dass** das mathematische Modell eine Übertragungsfunktion umfasst, mit deren Hilfe aus dem Ausgangssignal des Drucksensors (4) die die Klopffestigkeit charakterisierende Kenngröße berechnet wird.

3. Verfahren gemäß dem vorangegangenen Anspruch, **dadurch gekennzeichnet, dass** die Übertragungsfunktion als Differentialgleichung ausgebildet ist oder eine solche umfasst.

4. Verfahren gemäß dem vorangegangenen Anspruch, **dadurch gekennzeichnet, dass** die Differentialgleichung zeitliche Ableitungen unterschiedlicher Ordnung des Ausgangssignals des Drucksensors (4) bzw. des hieraus ermittelten Zylinderdrucks umfasst.

5. Verfahren gemäß dem vorangegangenen Anspruch, **dadurch gekennzeichnet, dass** die zeitlichen Ableitungen innerhalb der Differentialgleichung unterschiedlich stark gewichtet werden.

6. Verfahren gemäß einem der vorangegangenen Ansprüche, **dadurch gekennzeichnet, dass** mit Hilfe des mathematischen Modells der in der Norm ASTM D2699 vorgeschriebenen Pickup Sensor (5) simuliert wird, indem das Ausgangssignal des Drucksensors (4) derart korrigiert wird, dass das korrigierte Signal im Wesentlichen dem Ausgangssignal des in der Norm ASTM D2699 vorgeschriebenen Pickup Sensors (5) bei gleichem Zylinderdruck entspricht.

7. Verfahren gemäß einem der vorangegangenen Ansprüche, **dadurch gekennzeichnet, dass** mit Hilfe des mathematischen Modells die in der Norm ASTM D2699 beschriebene Messkette aus Pickup Sensor (5), Detonationmeter (7) und Knockmeter (8) simuliert wird.

8. Verfahren gemäß einem der vorangegangenen Ansprüche, **dadurch gekennzeichnet, dass** das Ausgangssignal des Drucksensors (4) mit Hilfe eines Tiefpassfilters gefiltert wird, und dass die die Klopffestigkeit des Kraftstoffs charakterisierende Kenngröße auf Basis des gefilterten Ausgangssignals berechnet wird.

9. Verfahren gemäß einem der vorangegangenen Ansprüche, **dadurch gekennzeichnet, dass** als Drucksensor (4) ein piezoelektrischer Drucksensor (4) zum Einsatz kommt.

10. Verfahren gemäß einem der vorangegangenen Ansprüche, **dadurch gekennzeichnet, dass** die Auswertung des Ausgangssignals des Drucksensors (4) unter Einsatz eines künstlichen neuronalen Netzes erfolgt.

11. Prüfanordnung zur Bestimmung einer die Klopffestigkeit charakterisierenden Kenngröße eines Kraftstoffs mit einem Prüfmotor (2), der wenigstens einen Zylinder (1) und einen Drucksensor (4) mit einer linearen Druck-Ausgangssignal-Kennlinie aufweist, wobei mit Hilfe des Drucksensors (4) der bei der Verbrennung des Kraftstoffs im Zylinder (1) herrschende Zylinderdruck detektierbar ist,
**dadurch gekennzeichnet,**
**dass** die Prüfanordnung eine Auswerteeinheit (6) umfasst, mit deren Hilfe basierend auf dem Ausgangssignal des Drucksensors (4) eine die Klopffestigkeit des Kraftstoffs charakterisierende Kenngröße berechenbar ist, wobei die Berechnung auf Basis eines mathematischen Modells erfolgt, welches die Abweichung der Druck-Ausgangssignal-Kennlinie des zum Einsatz kommenden Drucksensors (4) von der Druck-Ausgangssignal-Kennlinie eines in der Norm ASTM D2699 vorgeschriebenen Pickup Sensors (5) berücksichtigt.
